# EUROPEAN PATENT APPLICATION

(11) **EP 2 404 507 A1**
(43) Date of publication of application: **11.01.2012**
(21) Application number: 10168872.9
(22) Date of filing: 08.07.2010
(51) Int. Cl.: A23L 1/29, A23L 1/30, A61P 1/00, A61P 3/04, A61P 37/00

(54) **Array of age-tailored nutritional formula with optimum fat content**

(71) Applicant: Nestec S.A., 1800 Vevey (CH)
(72) Inventor: Magliola, Corinne, 1009, Pully (CH); Klassen, Petra Gerda, 1806, St. Legier (CH)
(74) Representative: Corticchiato, Olivier

(57) **Abstract**

The present invention relates to nutritional formulae which are specifically designed to address the needs of infants and young children between 0 and 2 years. In particular, the invention provides a set of nutritional compositions for infants and young children, each nutritional composition having age-specific fat contents. The set of the invention is specifically aimed at providing long-term benefits to an infant/young child by meeting its nutritional needs at each specific age.

## Description

### Field of the invention

The present invention relates to nutritional formulae which are specifically designed to address the needs of infants and young children between 0 and 2 years. In particular, the invention provides a set of nutritional compositions for infants and young children, each nutritional composition having age-specific fat contents. The set of the invention is specifically aimed at providing long-term benefits to an infant/young child by meeting its nutritional needs at each specific age.

### Background of the invention

Infant formulae, follow-up formulae and grown-up milks which are aimed at different age groups of 0 to 6 months, 6 months to 1 year and 1 year to 2 years respectively are well known. These formulae aim to meet the requirements of infants and young children at the different ages.

An age-tailored nutrition system for infants is described in WO 2009/068549, wherein the protein nature and content are adapted to specific age groups.

The recommended WHO guidelines and CODEX for infant formulae state that a minimum of 4.4 g/100kcal of fat should be present and not exceed 6 g/100kcal. For follow-up formulae, the recommendations state that the products shall not provide less than 3g fat per 100kcal and not more than 6g fat per 100kcal.

An article by Fuchs, G. J. et al. in Nutrition Research, 1996, vol. 16, issue 2, 391-400, found that infants from 6 months to 1 year fed a low-fat follow-up formula were not affected compared to those fed standard infant formula in terms of growth in weight, recumbent length or head circumference.

Opinions therefore diverge as to what the optimal fat content should be in infant and follow-up formulae.

Additionally, a number of follow-up formulae and grown-up milks are manufactured taking into account a hypothetical amount of complementary foods. Often however, the intake from complementary foods is over- or underestimated for specific age groups and an inadequate intake of certain nutrients results, leading to nutritional deficiencies in infants and young children.

Studies have shown that infants being fed with both infant formula and complementary foods may present deficiencies in certain nutrients, especially just after weaning and/or at the age of 6 months to 12 months.

It is therefore important to address these deficiencies and in particular to address the level of fat needed at specific ages in order to avoid overdosing fats at early ages and/or under-dosing fats at a later stage.

The composition of the human milk varies naturally according to the age of the infant. Such variations affect not only the overall caloric density but also greatly affect the relative composition of the milk in the diverse nutrients. For example, the fat composition and fat amount of human breast milk varies during breastfeeding according to the age of the infant.

It is therefore also important to develop infant and young child formulae which replicate human breast milk as far as possible in terms of nutritional properties.

Human breast milk is indeed the golden standard as for the composition of infant formula. Hence its composition should try to be mimicked to the extent possible for each age of the infant/young child.

### Object of the invention

The invention therefore aims at ensuring an optimal fat content in infant and young child formulae at all ages.

It is also an object of the invention to follow the nutritional evolution curve of human breast milk at all ages.

### Summary of the invention

The object is solved by means of the independent claims. The dependent claims further develop the central idea of the invention.

Thus, in a first aspect, the present invention relates to a set of nutritional compositions for infants and young children comprising at least
a first composition for infants between 0 and 6 months having a fat content of between 5 and 6g/100kcal, preferably between 5.1 and 5.8 g/100 kcal of the first composition,
a second composition for infants between 6 months and 1 year having a fat content of between 5 and 6g/100kcal, preferably between 5.5 and 6g/100 kcal of the second composition,
a third composition for young children from 1 year to 2 years having a fat content of between 5 and 6g/100kcal, preferably between 5.5 and 6g/100 kcal of the third composition,
a fourth composition for young children over 2 years having a fat content of between 3 and 5g/100kcal, preferably between 3.5 and 4.5g/100kcal of the fourth composition.

In a second aspect, the invention extends to a set of nutritional compositions for infants and young children, wherein said set comprises at least four nutritional compositions targeted at infants and young children between 0 and 6 months, between 6 months and 1 year, between 1 year and 2 years and above 2 years respectively, wherein said compositions have a higher than conventional fat content for infants between 6 months and 2 years and wherein at least the compositions targeted at infants between 0 and 6 months and between 6 months and 1 year are within at least 80%, preferably at least 90%, more preferably at least 95% of the average fat content of human breast milk at the corresponding ages.

The use of the sets of the present invention for feeding an infant/young child for at least the first two years of life or for providing an infant/young child with a balanced nutritional diet for at least the first two years of life is also part of the present invention.

In a further aspect, a method for providing nutrition to an infant/young child in at least the first two years of life comprising feeding to an infant/young child a set of nutritional compositions according to any of claims 1 to 8 at the corresponding ages is provided.

An age-tailored nutrition kit for infants and young children comprising a set of nutritional compositions according to any of claims 1 to 8, wherein the nutritional compositions are packed in single dose units, each unit comprising sufficient nutritional composition to prepare a single serving upon reconstitution with water also forms part of the invention.

The invention further pertains to an infant/young child nutrition regimen comprising feeding said infant between 0 to 6 months a nutritional composition having a fat content of between 5 and 6g/100kcal, preferably between 5.1 and 5.8 g/100 kcal, such that the total daily intake is at least 400 kcal, preferably at least 435 kcal, feeding said infant between 6 months and 1 year a nutritional composition having a fat content of between 5 and 6g/100kcal, preferably between 5.5 and 6g/100 kcal and complementary solid foods such that the total daily intake is at least 550 kcal, preferably at least 580 kcal, feeding said young child from 1 year to 2 years a nutritional composition having a fat content of between 5 and 6g/100kcal, preferably between 5.5 and 6g/100 kcal and complementary solid foods such that the total daily intake is at least 750 kcal, preferably at least 765 kcal, feeding said young child over 2 years a nutritional composition having a fat content of between 3 and 5g/100kcal, preferably between 3.5 and 4.5g/100kcal and complementary solid foods, such that the total daily intake is at least 900 kcal, preferably at least 1000 kcal.

Finally, another facet of the invention relates to a nutritional composition for young children between 1 year and 2 years comprising fat and long-chain unsaturated fatty acids, wherein the fat content is between 5 and 6g/100kcal, preferably between 5.5 and 6g/100 kcal and the long-chain unsaturated fatty acids are DHA and ARA.

### Detailed description of the invention

In the present specification, the following words are given a definition that must be taken into account when reading and interpreting the description, examples and claims.

Infant: according to the Commission Directive 91/321/EEC of 14 May 1991 on infant formulae and follow-on formulae, article 1.2 (a), the term "infants" means children under the age of 12 months. This definition is adopted in the present specification.

Young Children: according to the Commission Directive 91/321/EEC of 14 May 1991 on infant formulae and follow-on formulae, article 1.2 (b), the term "young children" means children aged between one and three years. This definition is adopted in the present specification.

Infant formulae: according to the Commission Directive 91/321/EEC of 14 May 1991 on infant formulae and follow-on formulae, article 1.2 (c), the term "infant formula" means foodstuffs intended for particular nutritional use by infants during the first four to six months of life and satisfying by themselves the nutritional requirements of this category of persons. This definition is adopted in the present specification. It has to be understood that infants can be fed solely with infant formulas, or that the infant formula can be used by the carer as a complement of human milk. It is synonymous to the widely used expression "starter formula".

Follow-on formulae: according to the Commission Directive 91/321/EEC of 14 May 1991 on infant formulae and follow-on formulae, article 1.2 (d), the term "follow-on formulae" means foodstuffs intended for particular nutritional use by infants aged over four months and constituting the principal liquid element in a progressively diversified diet of this category of persons. This definition is adopted in the present specification.

Probiotic: according to the paper Probiotics in Man and Animals, J. Appl Bacteriol. 66: 365-378, a probiotic is defined as a live microbial feed supplement which beneficially affects the host animal by improving its intestinal microbial balance.

The present invention relates to a set of nutritional compositions for infants and young children. The set is formed of at least four nutritional compositions aimed at at least four different age groups.

In the present invention, the nutritional compositions are preferably in the form of a powder to be reconstituted or concentrate to be diluted. The powder or concentrate can be reconstituted or diluted with water. The end product is thus preferably a liquid.

The set of the invention comprises a first composition for infants between 0 and 6 months having a fat content of between 5 and 6g/100kcal. Preferably, the fat content is between 5.1 to 5.8g/100kcal, most preferably it is between 5.3 and 5.7 g/100kcal.

The fat content of the first composition is preferably between 48 and 54%, preferably between 50 and 52% of the total energy for said first composition.

The set further comprises a second composition for infants between 6 months and 1 year having a fat content of between 5 and 6g/100kcal. Preferably, the fat content is between 5.5 and 6g/100 kcal, most preferably it is 5.6g/100kcal.

The fat content of the second composition is preferably between 48 and 54%, preferably between 50 and 54% of the total energy for said second composition.

The set further comprises a third composition for young children from 1 year to 2 years having a fat content of between 5 and 6g/100kcal. Preferably, the fat content is between 5.5 and 6g/100 kcal, most preferably it is 5.6g/100kcal.

The fat content of the third composition is preferably between 48 and 54%, preferably between 50 and 54% of the total energy for said third composition.

The set further comprises a fourth composition for young children over 2 years having a fat content of between 3 and 5g/100kcal. Preferably, the fat content is between 3.5 and 4.5g/100kcal, most preferably it is 4g/100kcal.

The fat content of the fourth composition is preferably between 35 and 51%, more preferably between 38 and 45%, most preferably between 35 and 40% of the total energy for said fourth composition.

The compositions which form part of the set according to the invention are thus characterized in that they provide a fat content which better meets the needs of infants/young children at the different ages. In particular, the fat content of the second composition for infants between 6 months and 1 year is higher than conventional compositions aimed for this age group. Also, the fat content of the third composition for young children between 1 year and 2 years is higher than conventional compositions for this age group.

Conventional compositions such as NAN® or Good Start® from Nestlé which are aimed at infants and young children of 6 months to 1 year and 1 year to 2 years generally have a fat content of 5.1 g fat per 100 Kcal or 3.4 g/100ml of ready to drink composition.

Thus, it has been found that the set of the invention provides long-term benefits over a period of at least two years, where the infants/young children have a balanced nutritional intake especially in terms of fat. The set provides an optimal fat content at all ages and avoids the pitfalls of overdosing or under-dosing fat.

It has further been found that the nutritional compositions of the present set work in synergy such that optimal health effects are observed when the nutritional compositions are used consequently. Therefore, using the nutritional compositions independently (i.e. not as part of a set) would not achieve the beneficial effects to the same extent.

Mimicking human breast milk to the best possible extent has been considered as a guidance as for the composition of the conventional infant at each individual age of the infant. However the present invention proposes to closely follow or get close to the composition of the human breast milk in the full array of individual compositions, comprising at least 4 specific stages (4 specific age groups). The array of compositions of the invention thus allows to more closely follow the composition of human breast milk by proposing to have 4 individual ages, whereas standard infant formula conventionally propose 2 or 3 stages.

Also, the array of compositions of the invention optimizes the fat intake of the infants during an extended period of time. It has been hypothesized from on-going related studies that this optimization can relate to long term health benefits such as lower occurrence of infections, improved immune function and immune defenses, lower occurrence of allergies, better gut functions, better gut maturation, optimum growth, avoidance of over-weight later in life and/or better brain development.

It is hypothesized that the optimized fat intake over time allows not only for the best caloric intake and best fatty acids profile and intake (for example fatty acid that have been shown to relate to brain development), but also that the fat enables the transport and intake of lipo-soluble nutrients which play an important role in growth and development. The fat acts indeed as a carrier to lipo-soluble nutrients. As an illustration, providing an optimized fat content in the compositions over a long period of time indirectly enhances the intake of lipo-soluble vitamins, or other nutrients (such as lipidic components of milk fat globule membranes) over an extended period of time.

The fat in the nutritional compositions may be selected from milk and/or vegetable fat. Typical vegetable fats include palm olein, high oleic sunflower oil, high oleic safflower oil, canola oil, fish oil, coconut oil, bovine milk fat or any mixtures thereof.

In a preferred embodiment, any of the nutritional compositions which form part of the set may further comprise long-chain polyunsaturated fatty acids (LC-PUFA). These have been linked to benefits in infant/young child development. Preferably, the LC-PUFA are selected from docosahexaenoic acid (DHA), arachidonic acid (ARA), eicosapentaenoic acid (EPA) or any mixtures thereof. Most preferably, the first, second and third nutritional compositions comprise a mixture of docosahexaenoic acid (DHA) and arachidonic acid (ARA). Most preferably, the fourth composition comprises docosahexaenoic acid (DHA).

The origin of DHA or ARA may be of importance in the context of the present invention as DHA and/or ARA from fish oil may have a different effect compared to microbial DHA or ARA. The sustained fat intake of the present invention may compensate for that effect.

Any of the compositions of the set of the invention may also comprise medium chain triglycerides and/or milk fat globule membranes (MFGM).

In the set of the present invention, any of the first, second, third or fourth composition may further comprise carbohydrates. Suitable carbohydrate sources include lactose, saccharose, maltodextrin, starch and mixtures thereof. In a preferred embodiment, the first and second compositions comprise lactose. Preferably, the amount of lactose in the first and second compositions is between 9.5 and 12 g/100 kcal, preferably between 10 and 11 g/100kcal. The third and fourth compositions preferably comprise a mixture of lactose and maltodextrin. Preferably, the maltodextrin has a DE of 19. Most preferably, the ratio of lactose to maltodextrin in the third and fourth compositions is 70:30.

The compositions may further comprise proteins such as intact or hydrolysed protein, proteins associated with milk fat globule membrane (MFGM), casein, whey, soy protein, or any mixtures thereof. In a preferred embodiment, the compositions comprise and mixture of whey and casein. Preferably, the ratio of whey to casein in the first composition is 70:30. The second and third compositions preferably have a ratio of whey:casein of 50:50. Preferably, the fourth composition has a whey to casein ratio of 40:60. The protein content in the compositions preferably varies between 1.5 to 2.5 g/100kcal. For instance, the first composition may comprise a protein content of 1.8 to 2.25 g/100kcal. The second composition may comprise a protein content of 1.8 g/100kcal. The third composition may comprise a protein content of 2 g /100kcal. The protein content of the fourth composition is preferably 2.25 g/100kcal.

Optionally, the compositions may comprise vitamins selected from vitamin A, beta-carotene, vitamin D, vitamin E, vitamin K1, vitamin C, vitamin B1, vitamin B2, niacin, vitamin B6, folic acid, pantothenic acid, vitamin B12, biotin, choline, inositol, taurine, carnitine, or any mixtures thereof.

Additionally, the compositions may comprise minerals selected from sodium, potassium, chloride, calcium, phosphorus, magnesium, manganese or any mixtures thereof.

Trace elements such as iron, iodine, copper, zinc, selenium, fluorine, chromium, molybdenum or any mixtures thereof may also be present in the compositions forming the set of the invention.

In a preferred embodiment, any of the compositions forming part of the set may comprise lactoferrin.

Additionally, any of the compositions may comprise prebiotics. If present, the prebiotic is preferably present in the compositions in an amount 1 to 20wt%, preferably 2 to 15wt% on a dry matter basis.

A prebiotic is a non-digestible food ingredient that beneficially affects the host by selectively stimulating the growth and/or activity of one or a limited number of bacteria in the colon, and thus improves host health. Such ingredients are non-digestible in the sense that they are not broken down and absorbed in the stomach or small intestine and thus pass intact to the colon where they are selectively fermented by the beneficial bacteria. Examples of prebiotics include certain oligosaccharides, such as fructooligosaccharides (FOS) and galactooligosaccharides (GOS). A combination of prebiotics may be used such as 90% GOS with 10% short chain fructo-oligosaccharides such as the product sold under the trade mark Raftilose® or 10% inulin such as the product sold under the trade mark Raftiline®.

A particularly preferred prebiotic is a mixture of galacto-oligosacch **aride(s)**, N-acetylated oligosaccharide(s) and sialylated oligosaccharide(s) in which the N- acetylated oligosaccharide(s) comprise 0.5 to 4.0% of the oligosaccharide mixture, the galacto-oligosaccharide(s) comprise 92.0 to 98.5% of the oligosaccharide mixture and the sialylated oligosaccharide(s) comprise 1.0 to 4.0% of the oligosaccharide mixture. This mixture is hereinafter referred to as "CMOS-GOS".

Preferably, any of the compositions of the set for the invention contain from 2.5 to 15.0 wt% CMOS-GOS on a dry matter basis with the proviso that the composition comprises at least 0.02 wt% of an N- acetylated oligosaccharide, at least 2.0 wt% of a galacto-oligosaccharide and at least 0.04 wt% of a sialylated oligosaccharide.

Suitable N-acetylated oligosaccharides include GalNAcαl, 3Galβ1, 4Glc and Galβ1, 6GalNAcαl, 3Galβ1, 4Glc. The N-acetylated oligosaccharides may be prepared by the action of glucosaminidase and/or galactosaminidase on N-acetyl-glucose and/or N-acetyl galactose. Bqually, N-acetyl-galactosyl transferases and/or N-acetyl-glycosyl transferases may be used for this purpose. The N-acetylated oligosaccharides may also be produced by fermentation technology using respective enzymes (recombinant or natural) and/or microbial fermentation. In the latter case the microbes may either express their natural enzymes and substrates or may be engineered to produce respective substrates and enzymes. Single microbial cultures or mixed cultures may be used. N-acetylated oligosaccharide formation can be initiated by acceptor substrates starting from any degree of polymerisation (DP) from DP=1 onwards. Another option is the chemical conversion of keto-hexoses (e.g. fructose) either free or bound to an oligosaccharide (e.g. lactulose) into N-acetylhexosamine or an N-acetylhexosamine containing oligosaccharide as described in Wrodnigg, T. M.; Stutz, A.E. (1999) Angew. Chem. Int. Ed. 38:827-828.

Suitable galacto-oligosaccharides include Galβ1, 6Gal, Galβ1,6Galβ1, 4Glc, Galβ1,6Galβ1,6Glc, Galβ1,3Galβ1,3Glc, Galβ1,3Galβ1,4Glc, Galβ1, 6Galβ1, 6Galβ1, 4Glc, Galβ1, 6Galβ1, 3Galβ1, 4Glc, Galβ1, 3Galβ1, 6Galβ1, 4Glc, Galβ1, 3Galβ1, 3Galβ1, 4Glc, Galβ1, 4Galβ1, 4Glc a n d Galβ1, 4Galβ1, 4Galβ, 4Glc.

Synthesised galacto-oligosaccharides such as Galβ1, 6Galβ1, 4Glc, Galβ, 6Galβ1, 6Glc, Galβ1, 3Galβ1, 4Glc, Galβ1, 6Galβ1, 6Galβ1, 4Glc, Galβ1, 6Galβ1, 3Galβ1, 4Glc and Galβ1, 3Galβ1, 6Galβ1, 4Glc, Galβ1, 4Galβ1, 4Glc **and** Galpl,4Galpl,4Galpl,4Glc and mixtures thereof are commercially available under the trademarks Vivinal® and Elix'or®. Other suppliers of oligosaccharides are Dextra Laboratories, Sigma-Aldrich Chemie GmbH and Kyowa Hakko Kogyo Co . , Ltd. Alternatively, specific glycosyltransferases, such as galactosyltransferases may be used to produce neutral oligosaccharides.

Suitable sialylaled oligosaccharides include NeuAcα2,3Galpl,4Glc and NeuAcα2, 6Galpl, 4Glc. These sialylated oligosaccharides may be isolated by chromatographic or filtration technology from a natural source such as animal milks. Alternatively, they may also be produced by biotechnology using specific sialyltransferases either by enzyme based fermentation technology (recombinant or natural enzymes) or by microbial fermentation technology. In the latter case microbes may either express their natural enzymes and substrates or may be engineered to produce respective substrates and enzymes. Single microbial cultures or mixed cultures may be used. Sialyl-oligosaccharide formation can be initiated by acceptor substrates starting from any degree of polymerisation (DP) from DP=1 onwards.

The compositions may optionally contain other substances which may have a beneficial effect such as nucleotides, nucleosides, and the like. Nucleotides may be selected from cytidine monophosphate (CMP), uridine monophosphate (UMP), adenosine monophosphate (AMP), guanosine monophosphate (GMP) or any mixtures thereof.

Any of the nutritional compositions may also comprise at least one probiotic bacterial strain.

Examples of suitable probiotic micro-organisms include yeasts such as *Saccharomyces, Debaromyces, Candida, Pichia* and *Torulopsis,* moulds such as *Aspergillus, Rhizopus, Mucor,* and *Penicillium* and *Torulopsis* and bacteria such as the genera *Bifidobacterium, Bacteroides, Clostridium, Fusobacterium, Melissococcus, Propionibacterium,* Streptococcus, *Enterococcus, Lactococcus,* Staphylococcus, *Peptostrepococcus, Bacillus, Pediococcus, Micrococcus, Leuconostoc, Weissella, Aerococcus*, *Oenococcus* and *Lactobacillus.* Specific examples of suitable probiotic micro-organisms are: *Saccharomyces cereviseae, Bacillus coagulans, Bacillus licheniformis, Bacillus subtilis, BifidobaCterium bifidum, Bifidobacterium infantis, Bifidobacterium longum, Enterococcus faecium, Enterococcus faecalis, Lactobacillus acidophilus*, *Lactobacillus alimentarius, Lactobacillus casei subsp. casei, Lactobacillus casei Shirota, Lactobacillus curvatus, Lactobacillus delbruckii subsp. lactis, Lactobacillus farciminus, Lactobacillus gasseri, Lactobacillus helveticus,* Lactobacillus *johnsonii, Lactobacillus reuteri, Lactobacillus rhamnosus (Lactobacillus GG), Lactobacillus sake, Lactococcus lactis, Micrococcus variants, Pediococcus acidilactici, Pediococcus pentosaceus, Pediococcus acidilactici, Pediococcus halophilus, Streptococcus faecalis, Streptococcus thermophilus, Staphylococcus carnosus,* and *Staphylococcus xylosus.*

Preferred probiotic bacterial strains include *Lactobacillus rhamnosus* ATCC 53103 obtainable from Valio Oy of Finland under the trade mark LGG, *Lactobacillus rhamnosus* CGMCC 1.3724, *Lactobacillus paracasei* CNCM 1-2116, *Lactobacillus reuteri* ATCC 55730 and *Lactobacillus reuteri* DSM 17938 obtainable from BioGaia AB, *Bifidobacterium lactis* CNCM 1-3446 sold *inter alia* by the Christian Hansen company of Denmark under the trade mark Bb 12 and *Bifidobacterium longum* ATCC BAA-999 sold by Morinaga Milk Industry Co. Ltd. of Japan under the trade mark BB536.

The amount of probiotic, if present, is preferably present in an amount of 10³ to 10¹² cfu/g, more preferably 10⁶ to 10¹¹ cfu/g, even more preferably 10⁴ to 10⁹ cfu/g, most preferably 10⁷ to 10⁹ cfu/g composition or per mL of composition. In a preferred embodiment, any of the nutritional compositions of the set comprise 2x10⁷ cfu/g. Most preferably, the probiotic is *Bifidobacterium lactis.*

In an embodiment, the amount of probiotics present in the compositions of the set may likewise preferably vary as a function of the age of the infant/young child.

The set of the invention aims to match closely the nutritional evolution curve of human breast milk, at least with respect to the fat content.

Thus, in an embodiment, each of the first, second, third or fourth composition of the set of the invention is within at least 80%, preferably at least 90%, more preferably at least 95% of the average fat content of human breast milk at the corresponding ages.

In a further embodiment, the invention covers a set of nutritional compositions for infants and young children, wherein said set comprises at least four nutritional compositions targeted at infants and young children between 0 and 6 months, between 6 months and 1 year, between 1 year and 2 years and above 2 years respectively, wherein said compositions have a higher than conventional fat content for infants between 6 months and 2 years and wherein at least the compositions targeted at infants between 0 and 6 months and between 6 months and 1 year are within at least 80%, preferably at least 90%, more preferably at least 95% of the average fat content of human breast milk at the corresponding ages. Preferably, each individual composition is within at least 80%, more preferably at least 90%, even more preferably at least 95% of the average fat content of human breast milk at the corresponding ages.

A nutritional composition for young children between 1 year and 2 years comprising fat and long-chain unsaturated fatty acids, wherein the fat content is between 5 and 6g/100kcal, preferably between 5.5 and 6g/100 kcal and the long-chain unsaturated fatty acids are DHA and ARA, also forms part of the invention. Such nutritional composition has a higher fat content than conventional nutritional composition for this age range. Since conventional nutritional compositions have been linked with deficiencies, the present composition thus provides a young child between 1 year and 2 years with an optimal fat range.

The nutritional compositions may be prepared in any suitable manner. For example, an infant formula may be prepared by blending together a protein source, a carbohydrate source, and a fat source in appropriate proportions. If used, emulsifiers may be included in the blend. The vitamins and minerals may be added at this point but are usually added later to avoid thermal degradation. Any lipophilic vitamins, emulsifiers and the like may be dissolved into the fat source prior to blending. Water, preferably water which has been subjected to reverse osmosis, may then be mixed in to form a liquid mixture.

The liquid mixture may then be thermally treated to reduce bacterial loads. For example, the liquid mixture may be rapidly heated to a temperature in the range of about 80°C to about 110°C for about 5 seconds to about 5 minutes. This may be carried out by steam injection or by heat exchanger; for example a plate heat exchanger. The liquid mixture may then be cooled to about 60°C to about 85°C for example by flash cooling. The liquid mixture may then be homogenised for example in two stages at about 7 MPa to about 40 MPa in the first stage and about 2 MPa to about 14 MPa in the second stage. The homogenised mixture may then be further cooled to add any heat sensitive components such as vitamins and minerals. The pH and solids content of the homogenised mixture are conveniently standardised at this point. The homogenised mixture is transferred to a suitable drying apparatus such as a spray drier or freeze drier and converted to powder. The powder should have a moisture content of less than about 3% by weight. Alternatively, the homogenized mixture is concentrated.

If it is desired to add probiotic(s), they may be cultured according to any suitable method and prepared for addition to the infant formula by freeze-drying or spray-drying for example. Alternatively, bacterial preparations can be bought from specialist suppliers such as Christian Hansen and Morinaga already prepared in a suitable form for addition to food products such as infant formula. Such bacterial preparations may be added to the powdered infant formula by dry mixing.

The set of the invention is used for feeding an infant/young child for at least the first two years of life. Thus, the invention also relates to a method for providing nutrition to an infant/young child in at least the first two years of life which comprises feeding to an infant/young child the set of nutritional compositions described herein at the corresponding ages.

In an embodiment of the invention, the nutritional compositions are packed in single dose units, each unit comprising sufficient nutritional composition to prepare a single serving upon reconstitution with water.

A single serving generally comprises 8 to 35 g, preferably 10 to 30g, most preferably 11 to 28 g of powder to be reconstituted with 80 to 300 mL, preferably between 100mL and 250 mL of water. Alternatively, if the nutritional composition is a concentrate, a single serving includes 1 to 50 mL of concentrate to be diluted with 50 to 250 mL of water.

The set of the invention is also used for providing an infant/young child with a balanced nutritional diet for at least the first two years of life.

Another facet of the invention therefore relates to an infant/young child nutrition regimen. The regimen comprises feeding an infant between 0 and 6 months a nutritional composition having a fat content having a fat content of between 5 and 6g/100kcal, preferably between 5.1 and 5.8 g/100 kcal, such that the total daily intake is at least 400 kcal, preferably at least 435 kcal.

It then comprises feeding said infant between 6 months and 1 year a nutritional composition having a fat content of between 5 and 6g/100kcal, preferably between 5.5 and 6g/100 kcal and complementary solid foods such that the total daily intake is at least 550 kcal, preferably at least 580 kcal.

Further, it comprises feeding said young child from 1 year to 2 years a nutritional composition having a fat content of between 5 and 6g/100kcal, preferably between 5.5 and 6g/100 kcal and complementary solid foods such that the total daily intake is at least 750 kcal, preferably at least 765 kcal.

Finally, it comprises feeding said young child over 2 years a nutritional composition having a fat content of between 3 and 5g/100kcal, preferably between 3.5 and 4.5g/100kcal and complementary solid foods, such that the total daily intake is at least 900 kcal, preferably at least 1000 kcal.

The complementary solid foods may be any of the foods commercially available for the corresponding age range. These include pureed vegetables, meats, fish, fruits, etc. It has been found that such a regimen provides a child with a balanced nutritional intake at least for the first two years of life and an optimal intake of fat at all ages.

The invention also pertains to an age-tailored kit for infants and young children. The kit comprises a set of nutritional compositions as described herein. The nutritional compositions are packed in single dose units, each unit comprising sufficient nutritional composition to prepare a single serving upon reconstitution with water. Typically, the units comprise 10 to 30 g of powdered nutritional composition or 5 to 50 g of a concentrate of nutritional composition. Preferably the units are in the form of capsules. They may also be in the form of stick packs or sachets.

The capsules may be disposable capsules equipped with opening means contained within the capsule to permit draining of the reconstituted formula directly from the capsule into a receiving vessel such as a young child bottle. Such a method of using capsules for dispensing an infant or young child nutritional composition is described in W02006/077259. The different nutritional compositions forming part of the set of the invention may be packed into individual capsules and presented to the consumer in multipacks containing a sufficient number of capsules to meet the requirements of an infant for one week for example. Suitable capsule constructions are disclosed in W02003/059778.

The present invention is further illustrated hereinafter by means of the following non-limiting examples.

### Examples

An age-tailored set of nutritional compositions are given in the table below:

| | Formula | | | | |
|---|---|---|---|---|---|
| | Age range | 0 to 6 months | 7th to 12 months | 1 to 2 yr | 2 to 3 yr |
| Basics | Reconstitutio n RTD Volume (ml) | 100 to 200 | 230 | 230 | 230 |
| | | | | | |
| | Energy density (kcal/100 ml) | 63-67 | 63 | 63 | 63 |
| Protein | Content (g/100kcal) | 1.8 - 2.25 | 1.8 | 2 | 2.25 |
| | Content (g/l) | 11.3 -15.1 | 11.3 | | |
| | Whey:Casein | 70:30 | 50:50 | 50:50 | 40:60 |
| | Functional proteins | . | | | - |
| Carbohydrates | Type | Lactose | Lactose | Lactose / MD DE19 (70:30) | Lactos e / MD DE19 (70:30 |
| | Content (g/100kcal) | 9.7 to 11.6 | 10.6 | 10.6 | 14.2 |
| | Content (g/1) | 65.0 to 73.5 | 66.8 | | |
| Lipids | Type | Milk & Veg. | Milk & Veg. | Milk & Veg. | Fat mix follow s AHA: sat. Fat <7%E + polyun s. <10%E LA/ALA 5.0 |
| | Content (g/100kcal) | 5.1 to 5.8 | 5.6 | 5.6 | 4 |
| | content (as % of total energy) | 45.9 to 52.2 | 45.8 | 50.4 | 50.4 |
| | Content (g/1) | 32.1 to 38.9 | 35.3 | | |
| | LC-PUFA | DHA + ARA | DHA + ARA | DHA + ARA | DHA 0.3%tf a |
| Soluble Fibers | Content (g/100kcal) | | | | |
| Probioti cs | Type | *B.lactis* | | | |
| | Content | 2x 10⁷cfu/g | | | |
| Nucleoti des | CMP (mg/100 kcal) | 1.1 | - | | - |
| | UMP | 0.7 | - | | - |
| | AMP | 0.7 | - | | - |
| | GMP | 0.2 | - | | - |
| | NucleoPremix | | - | | - |
| Minerals (/100kcal) | Na (mg) | 25 to 37.5 | 25 | 25 | - |
| | K (mg) | 80 to 95 | 80 | 80 | - |
| | *Na*/*K (molar ratio)* | *0.53 to 0.67* | 0.53 | 0.53 | - |
| | *(Na +K)* /*Cl molar ratio* | *1.71 to 181* | *1.71* | *1.71* | - |
| | Cl (mg) | 65 to 80 | 65 | 65 | - |
| | Ca (mg) | 60 | 60 | 80 | 80 |
| | P (mg) | 33 | 33 | 50 | 50 |
| | Mg (mg) | 7 | 7 | 10 | 10 |
| | Mn (µg) | 5 | 5 | - | - |
| | Ca/P | 1.8 | 1.8 | 1.6 | 1.6 |
| Vitamins (/100kcal) | Vit. A (mg RE) | 0.09 to 01125 | 0.09 | 0.06 | 0.06 |
| | Beta carotene (µg) | | | | |
| | Vit. D (mg) | 0.0015 | 0.0015 | 0.0018 | 0.0018 |
| | Vit. E (mg) | 1.3 | 1.3 | 1.3 | 1.3 |
| | Vit . K1 (µg) | 8 | 8 | 4 | 4 |
| | Vit. C (mg) | 15 | 15 | 10 | 10 |
| | Vit. B1 (mg) | 0.07 to 0.1 | 0.1 | 0.08 | 0.08 |
| | Vit. B2 (mg) | 0.1 | 0.1 | 0.08 | 0.08 |
| | Niacin (mg) | 0.5 | 0.5 | 0.8 | 0.8 |
| | Vit. B6 (mg) | 0.05 | 0.05 | 0.07 | 0.07 |
| | Folic acid (µg) | 15 to 16 | 15 | 15 | 15 |
| | Pantothenic Acid (mg) | 0.7 to 0.8 | 0.8 | 0.4 | 0.4 |
| | Vit . B12 (µg) | 0.2 | 0.2 | 0.15 | 0.15 |
| | Biotin (µg) | 2 | 2 | 1.5 | 1.5 |
| | Choline (mg) | 20 | 20 | 30 | 30 |
| | Inositol (mg) | 25 | 20 | - | - |
| | Taurine (mg) | 8 | 6 | - | - |
| | Carnitine (mg) | 1.5 | - | - | - |
| Trace Elements (/100kcal) | Fe (mg) | 0.7 | 1 | 1 | 1 |
| | I (µg) | 15 to 20 | 20 | 15 | 15 |
| | Cu (mg) | 0.06 to 0.08 | 0.06 | 0.05 | 0.05 |
| | Zn (mg) | 1 to 1.2 | 0.8 | 0.6 | 0.6 |
| | Se (µg) | 3 to 4 | 3 | 3.5 | 3.5 |
| | F (µg) | | - | - | - |

It was shown that feeding a set of nutritional compositions as exemplified above, the dietary need of infants/young children were met, in particular with regards to fat. Also, the compositions were a close match of human breast milk at the different ages.

## Claims

1. A set of nutritional compositions for infants and young children comprising at least
a first composition for infants between 0 and 6 months having a fat content of between 5 and 6g/100kcal, preferably between 5.1 and 5.8 g/100 kcal of the first composition,
a second composition for infants between 6 months and 1 year having a fat content of between 5 and 6g/100kcal, preferably between 5.5 and 6g/100 kcal of the second composition,
a third composition for young children from 1 year to 2 years having a fat content of between 5 and 6g/100kcal, preferably between 5.5 and 6g/100 kcal of the third composition,
a fourth composition for young children over 2 years having a fat content of between 3 and 5g/100kcal, preferably between 3.5 and 4.5g/100kcal of the fourth composition.

2. Set according to claim 1, wherein the fat content of the first composition is between 48 and 54% of the total energy for said first composition,
wherein the fat content of the second composition is between 48 and 54% of the total energy for said second composition,
wherein the fat content of the third composition is between 48 and 54% of the total energy for said third composition,
wherein the fat content of the fourth composition is between 35 and 51%, preferably between 38% and 45% of the total energy for said fourth composition.

3. Set according to any of the preceding claims, wherein any of the first, second, third or fourth composition comprises long-chain polyunsaturated fatty acids selected from docosahexaenoic acid(DHA), arachidonic acid (ARA), or eicosapentaenoic acid (EPA), and/or comprises medium-chain triglycerides and/or milk fat globule membranes (MFGM), and any mixtures thereof.

4. Set according to any of the preceding claims, wherein any of the first, second, third or fourth composition comprises vitamins, minerals, trace elements, lactoferrin, probiotics, prebiotics, proteins associated with milk fat globule membrane (MFGM), hydrolysed or non-hydrolysed protein, carbohydrates such as lactose, maltodextrin, and any mixtures thereof.

5. Set according to any of the preceding claims, wherein each of the first, second, third and/or fourth composition is within at least 80%, preferably at least 90%, more preferably at least 95% of the average fat content of human breast milk at the corresponding ages.

6. Set according to any of the preceding claims, wherein the nutritional compositions are in the form of a powder to be reconstituted or a concentrate to be diluted.

7. Set of nutritional compositions for infants and young children, wherein said set comprises at least four nutritional compositions targeted at infants and young children between 0 and 6 months, between 6 months and 1 year, between 1 year and 2 years and above 2 years respectively, wherein said compositions have a higher than conventional fat content for infants between 6 months and 2 years and wherein at least the compositions targeted at infants between 0 and 6 months and between 6 months and 1 year are within at least 80%, preferably at least 90%, more preferably at least 95% of the average fat content of human breast milk at the corresponding ages.

8. Set according to claim 7, wherein said set comprises at least four nutritional compositions targeted at infants and young children between 0 and 6 months, between 6 months and 1 year, between 1 year and 2 years and above 2 years respectively, wherein said compositions have a higher than conventional fat content for infants between 6 months and 2 years and wherein each individual composition is within at least 80%, preferably at least 90%, more preferably at least 95% of the average fat content of human breast milk at the corresponding ages.

9. Set according to any of the preceding claims, for use in feeding an infant for at least the first two years of life.

10. Set according to any of the preceding claims, for use in providing an infant with a balanced nutritional diet for at least the first two years of life.

11. Method for providing nutrition to an infant in at least the first two years of life comprising feeding to an infant a set of nutritional compositions according to any of claims 1 to 8 at the corresponding ages.

12. Method according to claim 11, wherein the nutritional compositions are packed in single dose units, each unit comprising sufficient nutritional composition to prepare a single serving upon reconstitution with water.

13. An age-tailored nutrition kit for infants and young children comprising a set of nutritional compositions according to any of claims 1 to 8, wherein the nutritional compositions are packed in single dose units, preferably capsules, each unit comprising sufficient nutritional composition to prepare a single serving upon reconstitution with water.

14. An infant/young child nutrition regimen comprising feeding said infant between 0 to 6 months a nutritional composition having a fat content of between 5 and 6g/100kcal, preferably between 5.1 and 5.8 g/100 kcal, such that the total daily intake is at least 400 kcal, preferably at least 435 kcal feeding said infant between 6 months and 1 year a nutritional composition having a fat content of between 5 and 6g/100kcal, preferably between 5.5 and 6g/100 kcal and complementary solid foods such that the total daily intake is at least 550 kcal, preferably at least 580 kcal,
feeding said young child from 1 year to 2 years a nutritional composition having a fat content of between 5 and 6g/100kcal, preferably between 5.5 and 6g/100 kcal and complementary solid foods such that the total daily intake is at least 750 kcal, preferably at least 765 kcal,
feeding said young child over 2 years a nutritional composition having a fat content of between 3 and 5g/100kcal, preferably between 3.5 and 4.5g/100kcal and complementary solid foods, such that the total daily intake is at least 900 kcal, preferably at least 1000 kcal.

15. Nutritional composition for young children between 1 year and 2 years comprising fat and long-chain unsaturated fatty acids, wherein the fat content is between 5 and 6g/100kcal, preferably between 5.5 and 6g/100 kcal and the long-chain unsaturated fatty acids are DHA and ARA.
